## Europäisches Patentamt

## European Patent Office

## Office européen des brevets

(19)

(11) Veröffentlichungsnummer: **0 103 210**
**B1**

(12) **EUROPÄISCHE PATENTSCHRIFT**

(45) Veröffentlichungstag der Patentschrift:
**25.11.87**

(21) Anmeldenummer: **83108199.7**

(22) Anmeldetag: **19.08.83**

(51) Int. Cl.⁴: **C 12 N 9/04**, C 12 P 7/40,
C 12 P 11/00 //
C12R1/225

(54) L-2-Hydroxy-4-methylpentansäure-Dehydrogenase, Verfahren zu ihrer Gewinnung und ihre Verwendung.

(30) Priorität: **14.09.82 DE 3234022**

(43) Veröffentlichungstag der Anmeldung:
**21.03.84 Patentblatt 84/12**

(45) Bekanntmachung des Hinweises auf die Patenterteilung:
**25.11.87 Patentblatt 87/48**

(84) Benannte Vertragsstaaten:
**BE CH DE FR GB LI NL SE**

(56) Entgegenhaltungen:
EP-A-0 000 560
DE-B-2 841 414
US-A-4 326 031

CHEMICAL ABSTRACTS, Band 76, Nr. 17, 24. April
1972, S. 223-224, Nr. 96726j, Columbus, Ohio, US, M.
SHARPE u.a.: "Slime-forming heterofermentative
species of the genus Lactobacillus"
CHEMICAL ABSTRACTS, Band 99, Nr. 23, 5.
Dezember 1983, S. 609-610, Nr. 193133w, Columbus,
Ohio, US, W. HUMMEL u.a.: "Large scale
production of D-lactate dehydrogenase for the
stereospecific reduction of pyruvate and
phenylpyruvate"

(73) Patentinhaber: **Degussa Aktiengesellschaft,
Weissfrauenstrasse 9, D-6000 Frankfurt am Main
1 (DE)**
Patentinhaber: **Gesellschaft für Biotechnologische
Forschung mbH (GBF), Mascheroder Weg 1,
D-3300 Braunschweig- Stöckheim (DE)**

(72) Erfinder: **Leuchtenberger, Wolfgang, Dr. Dipl.-
Chem., Geschwister- Scholl- Strasse 1, D-6454
Bruchköbel (DE)**
Erfinder: **Kula, Maria- Regina, Dr., Forstweg 15,
D-3340 Wolfenbüttel (DE)**
Erfinder: **Hummel, Werner, Dr., Glückstrasse 3,
D-3300 Braunschweig (DE)**
Erfinder: **Schütte, Horst, Nordring 29a, D-3320
Salzgitter 51 (DE)**

## Beschreibung

Gegenstand der Erfindung ist eine L-2-Hydroxy-4-methylpentansäure-Dehydrogenase, gekennzeichnet durch die folgenden Eigenschaften:

a) spezifische Aktivität gegenüber L-2-Hydroxy-4-methylpentansäure,
b) abhängig von Nicotinamid-adenin-dinucleotid (NAD),
c) Temperatur-Optimum der Wirkung 40 bis 60°C,
d) Temperatur-Optimum der Stabilität $\leqslant$ 40°C,
e) pH-Optimum für die Dehydrogenierungsreaktion 8,0 bis 8,5,
f) pH-Optimum für die Reduktionsreaktion um 7,0,
g) pH-Stabilitätsbereich 6,5 bis 8,5,
h) Michaelis-Konstante ($K_M$-Wert) gegenüber L-2-Hydroxy-4-methylpentansäure 0,62 x $10^{-3}$M und
i) zusätzliche Aktivität gegenüber L-2-Hydroxy-pentansäure, L-2-Hydroxyhexansäure und L-2-Hydroxy-4-(methylmercapto)-buttersäure
und erhältlich durch Züchtung des Stammes Lactobacillus confusus DSM 20196 und Gewinnung daraus.

Ein weiterer Gegenstand der Erfindung ist ein Verfahren zur Gewinnung der neuen L-2-Hydroxy-4-methylpentansäure-Dehydrogenase, welches dadurch gekennzeichnet ist, daß man den Stamm Lactobacillus confusus DSM 20196 in einem wässrigen Nährmedium züchtet, das eine Quelle für Kohlenstoff und Stickstoff, sowie Mineralsalze, Wuchsstoffe und Vitamine enthält und zu Beginn der Züchtung einen pH von 6,5 aufweist, nach beendeter Züchtung die Zellen durch Zentrifugation erntet, sie in einer auf pH 7 gepufferten Suspension aufschließt und das Enzym aus dem Extrakt gewinnt.

Gegenstand der Erfindung ist schließlich auch die Verwendung des neuen Enzyms zur enzymatischen Umwandlung von L-2-Hydroxy -4-methylpentansäure, L-2-Hydroxypentansäure, L-2-Hydroxy-hexansäure oder L-2-Hydroxy-4-(methylmercapto)-buttersäure in die entsprechende 2-Ketocarbonsäure bzw. von 2-Keto-4-methylpentansäure, 2-Ketopentansäure, 2-Ketohexansäure oder 2-Keto-4-(methylmercapto)-buttersäure in die entsprechende L-2-Hydroxycarbonsäure.

Das neue Enzym weist eine spezifische Aktivität gegenüber L-2-Hydroxy-4-methylpentansäure und eine zusätzliche Aktivität gegenüber verschiedenen anderen L-2-Hydroxycarbon-säuren, insbesondere gegenüber L-2-Hydroxypentansäure, L-2-Hydroxyhexansäure und L-2-Hydroxy-4-(methylmercapto)-buttersäure, auf. Es ist Coenzym-abhängig und bedarf für die Dehydrogenierungsreaktion der Gegenwart von Nicotinamid-adenin-dinucleotid ($NAD^+$) und für die Gegenreaktion, also die Hydrierung der jeweiligen 2-Ketocarbonsäure, der Gegenwart des hydrierten Nicotinamid-adenin-dinucleotids (NADH). Das Temperatur-Optimum der Wirkung liegt in dem Bereich zwischen 40 und 60°C, das Temperatur-Optimum der Stabilität bei maximal etwa 40°C, das pH-Optimum für die Dehydrogenierungsreaktion bei etwa 8,0 bis 8,5 und für die Gegenreaktion bei etwa 7,0. Das neue Enzym ist am stabilsten im pH-Bereich zwischen etwa 6,5 und 8,5. Die Michaelis-Konstante ($K_M$-Wert) für das Substrat L-2-Hydroxy-4-methylpentansäure beträgt 0,62 x $10^{-3}$M.

Das neue Enzym kann aus einem bei der Deutschen Sammlung von Mikroorganismen in Göttingen erhältlichen Mikroorganismus, nämlich einem Stamm Lactobacillus confusus (Katalognummer DSM 20196), gewonnen werden. Die Züchtung des Mikroorganismus erfolgt zweckmäßigerweise in dem im Katalog der Deutschen Sammlung von Mikroorganismen oder in dem nachfolgend angegebenen Nährmedium. Im folgenden wird die Gewinnung des neuen Enzyms aus dem Stamm Lactobacillus confusus DSM 20196 beschrieben:

1. Züchtung des Mikroorganismus:
Zur Züchtung wird der Stamm Lactobacillus confusus DSM 20196 in folgendem Medium angezogen:

| | | |
|---|---|---|
| Glukose | 20 | g |
| Hefeextrakt | 10 | g |
| Fleischextrakt | 0,5 | g |
| Natriumacetat | 5 | g |
| $K_2HPO_4$ | 2 | g |
| $M_gSO_4$ | 0,2 | g |
| $MnSO_4$ | 0,05 | g |

deionisiertes Wasser auf 1 l

Der pH-Wert dieser Lösung wird auf 6,5 eingestellt, dann wird für 15 Minuten bei 121°C (2 bar) sterilisiert.

Zur Anzucht werden 5 ml Medium im Reagenzglas mit einer Öse voll Lactobacillus confusus vom Stichagar-Röhrchen beimpft und 16 bis 20 Stunden bei 30°C bebrütet. 4 ml der gewachsenen Kultur werden dann als Animpfkultur für 200 ml Medium (in 500 ml-Erlenmeyer-Kolben) verwendet.

Nach 20 bis 24 Stunden werden diese 200 ml als Vorkultur für einen 10 l-Fermenter verwendet, damit können dann 200 l und in der Folge 5.000 l angeimpft werden. Der pH-Wert, der im Verlauf des Wachstums absinkt, wird in den Fermentern mit konzentriertem Ammoniak bei 5,0 gehalten. Gegen Ende der logarithmischen Wachstumsphase wird die Kultur abgekühlt und durch Zentrifugation werden die Zellen geerntet. Die Biomasse kann bei -20°C ohne größere Verluste an Aktivität für einige Wochen/Monate zwischengelagert werden.

2. Gewinnung und Reinigung des Enzyms:
a) Rohextrakt



0 103 210

24 kg Lactobacillus confusus-Zellen (Feuchtmasse) werden in 100 mM Phosphatpuffer für pH 7,0, der 0,1 % (v/v) 2-Mercaptoethanol enthält, suspendiert. Das Endvolumen von 60 l entspricht einer etwa 40 %igen Zellsuspension. Die Zellen werden in einer kontinuierlich arbeitenden Industrie-Glasperlen-Kugelmühle (NETZSCH LME 20) aufgeschlossen. Der horizontal angeordnete Mahlbehälter mit einem Fassungsvermögen von 22,7 l wird mit 0,55 bis 0,85 mm Glasperlen gefüllt, so daß sich ein Schüttelvolumen von 19,3 l ergibt (85 %). Der Aufschluß wird bei einer Rührwellendrehzahl von 1200 UPM und einer Durchflußrate von 100 l/h durchgeführt. Der Kühlmantel des Mahlbehälters wird während des Laufes mit Ehtylenglycol-Lösung von -10°C gekühlt, um eine Erwärmung des Produkts weitgehend zu vermeiden. Nach zwei Durchläufen ist ein Desintegrationsgrad von etwa 90 % erreicht. Der pH-Wert der Suspension fällt während der Homogenisation auf 6,3 ab.

b) Flüssig-Flüssig-Verteilung

Mit dem ersten Verteilungsschritt sollen die Zellbruchstücke aus dem Rohextrakt abgetrennt werden. Zu diesem Zweck wird ein wässriges 2-Phasensystem hergestellt, welches 18 % (w/w) Polyethylenglycol 1500, 7 % (w/w) Phosphatpuffer für pH 7,0 und 60 l Rohextrakt in einem 120 kg-System enthält. Um eine gute Verteilung zu erreichen, wird das 2-Phasen-System 2 Stunden lang gerührt und anschließend mit einem Tellerseparator (Typ Gyrotester B von α-Laval) separiert. Die Durchflußrate während der Trennung der Phasen beträgt 60 l/h, es werden 4 Regulierschrauben von 13,5 mm eingesetzt. Die Oberphase (86 l) enthält praktisch die gesamte Aktivität (Ausbeute 98,4 %) an L-2-Hydroxy-4-methylpentansäure-Dehydrogenase. Die Unterphase enthält die Zellbruchstücke und wird verworfen.

Die enzymhaltige Oberphase des vorhergehenden Schrittes wird mit 2 % (w/v) Polyethylenglycol 10 000, 10 % (w/v) Phosphatpuffer für pH 6,0 und 0,2 M Natriumchlorid, berechnet auf ein Endvolumen von 172 l, versetzt und 2 Stunden gerührt. Das sich ausbildende Polyethylenglycol/Salz-System läßt man in einem Glasbehälter absetzen, die Trennung ist nach etwa 1 Stunde vollständig. Bei diesem Verteilungsschritt werden sehr viele Proteine in die Unterphase extrahiert, unter anderem der größte Anteil der im Überschuß vorhandenen D-Lactat-Dehydrogenase, während die L-2-Hydroxy-4-me-thylpentansäure-Dehydrogenase weitgehend in der Oberphase (71 l) verbleibt. Die Trennung der Phasen erfolgt durch Ablaufenlassen der Unterphase.

Die Oberphase das vorhergehenden Schrittes wird mit 10 % (w/v) Phosphatpuffer für pH 6,0 und 0,2 M Natriumchlorid versetzt und dann auf 142 l aufgefüllt. Nach einer Rührzeit von 2 Stunden läßt man die Phasen sich in einem Absetztank trennen. Die L-2-Hydroxy-4-methylpentansäure-Dehydrogenase-Ak-tivität befindet sich nunmehr in der Unterphase (82 l).

c) Diafiltration

Die Unterphase wird in einer Romicon Hohlfaserpatrone (Typ HF 30-20-GM-80) konzentriert und durch Zugabe von Phosphatpuffer für pH 6,5 auf eine Endkonzentration von 200 mM diafiltriert. Anschließend wird mit einer Amicon-Hohlfaserpatrone (HI P30) die Enzymlösung auf 500 ml konzentriert.

d) DEAE-Cellulose-Chromatographie

Das konzentrierte und diafiltrierte Enzym wird auf eine 5 x 40 cm Säule aufgepumpt, die mit Whatman-Cellulose DE 52 gepackt ist. Die DEAE-Cellulose ist gegen einen Puffer equilibriert worden, der 200 mM Phosphatpuffer für pH 6,5 und 0,1 % (v/v) 2-Mercaptoethanol enthält. Die Säule wird zunächst mit 2,5 l Startpuffer nachgewaschen und das Enzym anschließend mit einem linearen Gradienten (2 x 2 l) von 0 bis 1 M Natriumchlorid in Startpuffer eluiert. Die L-2-Hydroxy-4-methylpentansäure-De-hydrogenase eluiert mit 0,3 M Natriumchlorid und wird vollständig von den restlichen Anteilen der D-Lactat-Dehydrogenase abgetrennt. Die aktiven Fraktionen werden durch Ultrafiltration konzentriert und bei 4°C aufbewahrt. Die Aufreinigungsschritte sind in Tabelle 1 zusammengefaßt.

**Tabelle 1**

Reinigung von L-2-Hydroxy-4-methylpentansäure-Dehydrogenase

| Reinigungsschritt | Volumen l | Protein g | Totalaktivität U | Spez. Aktivität U.mg$^{-1}$ | Ausbeute % | Anreicherung -fach |
|---|---|---|---|---|---|---|
| Rohextrakt | 60 | 1518 | 264 000 | 0,17 | 100 | 1 |
| Oberphase I | 86 | 955 | 259 700 | 0,27 | 98,4 | 1,6 |
| Oberphase II | 71 | 70 | 172 500 | 2,46 | 65,3 | 14,5 |
| Unterphase III | 82 | 48 | 159 800 | 3,33 | 60,5 | 19,6 |
| Diafiltration | 0,5 | 26 | 133 000 | 5,12 | 50,4 | 30,1 |
| DEAE-Cellulose | 0,95 | 3,6 | 107 000 | 30,1 | 40,5 | 177,1 |

Das Molekulargewicht der L-2-Hydroxy-4-methylpentansäure-Dehydrogenase wurde durch Gelfiltration an Sephacryl S 300 superfine zu etwa 125 000 ± 15 000 Dalton bestimmt. Die spezifische Aktivität des Enzyms wurde bei der Gelfiltration auf Werte über 100 U.mg$^{-1}$ verbessert.

Im nachfolgenden Beispiel 1 wird die Aktivität einer angereicherten L-2-Hydroxy-4-methylpentansäure-Dehydrogenase-Präparation für die Umwandlung verschiedener 2-Hy-droxycarbonsäuren in die entsprechenden 2-Ketocarbonsäu-ren geprüft. Gemessen wird jeweils die maximale Anfangs-Reaktionsgeschwindigkeit $V_{max}$ und der $K_M$-Wert.

3

**Beispiel 1**

Die Reaktionsgeschwindigkeit für die Dehydrogenierung von L-2-Hydroxy-4-methylpentansäure wurde in folgendem Testansatz untersucht: 0,099 M Phosphatpuffer für pH 8,0, 3 mM NAD$^+$ und limitierende Mengen an Enzym. Die Konzentration der L-2-Hydroxy-4-methylpentansäure wurde im Bereich von 0,2 bis 10 mM variiert. Die Zunahme der Extinktion durch das entstehende NADH wurde bei 340 nm gemessen. Abgezogen wurde ein Nullwert, den man erhält, wenn der Test ohne L-2-Hydroxy-4-methylpentansäure abläuft. Die Enzymaktivität wird in internationalen Einheiten U (Units) angegeben, wobei ein U die Entstehung von 1 µMol NADH/min und ml bedeutet. In entsprechender Weise wurde die Enzymaktivität gegenüber anderen 2-Hydroxycarbonsäuren bestimmt. Die Konzentration der entsprechenden 2-Hydroxycarbonsäuren im Test wurde zwischen 0,2 und maximal 100 mM variiert. Durch Steigerung der Substratkonzentration wurde die maximale Anfangsreaktionsgeschwindigkeit $V_{max}$ ermittelt. Die Michaelis-Konstante ($K_M$-Wert) entspricht der Substratkonzentration in Mol/Liter, bei der die Reaktionsgeschwindigkeit V die Hälfte der maximalen Anfangsreaktionsgeschwindigkeit beträgt. Die kinetischen Konstanten $V_{max}$ und $K_M$ wurden durch nicht-lineare Regression der Michaelis-Menten-Gleichung ermittelt und sind in Tabelle 2 zusammengefaßt.

Die maximale Anfangsreaktionsgeschwindigkeit $V_{max}$ mit der Dimension $\mu$Mol x min$^{-1}$ x mg$^{-1}$ ergibt sich durch Division der Volumenaktivität (U/ml), die bei Substratsättigung (in der Regel 10 x $K_M$, sonst besonders angegeben) gemessen wurde, durch den Proteingehalt der eingesetzten Enzymlösung in mg/ml.

**Tabelle 2**

| Substrat | maximale Anfangs-Reakt.-Geschw. ($\mu$Mol x min$^{-1}$ x mg$^{-1}$) | $V_{max}$ $K_M$-Wert* (M) |
|---|---|---|
| L-2-Hydroxy-4-methylpentansäure | 9,8 | 0,62 x 10$^{-3}$ |
| DL-2-Hydroxy-4-methylpentansäure | 9,6 | 1,4 x 10$^{-3}$ |
| DL-2-Hydroxypentansäure | 7,9 (6 x $K_M$) | 3,55 x 10$^{-3}$ |
| DL-2-Hydroxyhexansäure | 11,8 (5 x $K_M$) | 4,5 x 10$^{-3}$ |
| DL-2-Hydroxy-4-(methylmercapto)-buttersäure | 1,2 | 11,0 x 10$^{-3}$ |

* bei den Enantiomeren-Gemischen wurde der Berechnung die Gesamtkonzentration zugrundegelegt.

Die Tabelle zeigt, daß eine Reihe von 2-Hydroxycarbon-säuren zur 2-Ketocarbonsäure oxidiert werden. Beim Vergleich der für das reine L-Enantiomere und das D,L-Gemisch gefundenen Konstanten fällt auf, daß die D-Komponente im Bereich der gemessenen Konzentration keinen Einfluß auf die kinetischen Konstanten hat. Bei 100 mM Substratkonzentration wird dagegen bereits eine deutliche Substratüberschuß-Inhibierung bemerkt (Tabelle 3).

Die in Tabelle 3 angegebenen relativen Aktivitäten (U/ml) wurden bei Substrat-Endkonzentrationen von 1 mM, 10 mM und 100 mM gemessen. Es wurde in allen Fällen die gleiche Enzymlösung eingesetzt.

**Tabelle 3**

| Substrat | Testkonzentration | | |
|---|---|---|---|
| | 1 mM (U/ml) | 10 mM (U/ml) | 100 mM (U/ml) |
| L-2-Hydroxy-4-methylpentansäure | 28,4 | 49,7 | 5,0 |
| DL-2-Hydroxy-4-methylpentansäure | 16,6 | 39,8 | 2,9 |
| DL-2-Hydroxypentansäure | 6,2 | 31,2 | 5,3 |
| DL-2-Hydroxyhexansäure | 8,4 | 39,5 | 3,9 |
| DL-2-Hydroxy-4-(methylmercapto)-buttersäure | 0,46 | 2,7 | 5,1 |

Die Abhängigkeit der Reaktionsgeschwindigkeit von der NAD$^+$-Konzentration wurde in einem Testansatz ermittelt, der 0,1 M Phosphatpuffer für pH 8,0, 6,3 mM L-2-Hydroxy-4-methylpentansäure und limitierende Mengen an Enzym enthielt. Die NAD$^+$-Konzentration wurde im Bereich von 0,05 bis 7,0 mM variiert.

Der $K_M$-Wert für NAD$^+$ wurde durch nicht-lineare Regression der Michaelis-Menten-Gleichung ermittelt und beträgt 3,3 x 10$^{-4}$ M.

Im nachfolgenden Beispiel 2 wird die Aktivität einer angereicherten L-2-Hydroxy-4-methylpentansäure-Dehydrogenase-Präparation für die Umwandlung verschiedener 2-Ke-tocarbonsäuren in die entsprechenden 2-Hydroxycarbonsäuren überprüft. Gemessen wird jeweils wieder die maximale Anfangsreaktionsgeschwindigkeit $V_{max}$ und der $K_M$-Wert.

**Beispiel 2**

Die Reaktionsgeschwindigkeit für die Umsetzung von 2-Keto-4-methylpentansäure zu L-2-Hydroxy-4-methylpentansäure wurde in folgendem Testansatz ermittelt: 0,1 M Phosphatpuffer für pH 7,0, 0,235 mM NADH und limitierende Mengen an Enzym. Die Konzentration der 2-Keto-4-methylpentansäure wurde im Bereich von 0,01 bis 10 mM variiert. Die Reaktion wurde mit einem photometrischen Test verfolgt, in dem die Abnahme der Extinktion von NADH bei 340 nm gemessen wird. Abgezogen wurde ein Nullwert, den man erhält, wenn der Test ohne 2-Keto-4-methylpentansäure abläuft. Die Enzymaktivität wird in internationalen Einheiten (Units) angegeben, wobei ein U den Verbrauch von 1 µM NADH/min und ml bedeutet. In entsprechender Weise wurde die Enzymaktivität gegenüber anderen 2-Ketocarbonsäuren bestimmt. Die Ketosäure-Konzentration wurde jeweils im Bereich von 0,01 bis maximal 10 mM variiert. Die maximale Anfangsreaktionsgeschwindigkeit wurde durch die Steigerung der Substratkonzentration ermittelt. Die Michaelis-Konstante ($K_M$-Wert) entspricht der Substratkonzentration in Mol/Liter, bei der die Reaktionsgeschwindigkeit V die Hälfte der maximalen Anfangsreaktionsgeschwindigkeit beträgt. Die kinetischen Konstanten $V_{max}$ und $K_M$ wurden durch nicht-lineare Regression der Michaelis-Menten-Gleichung ermittelt und sind in der nachfolgenden Tabelle 4 zusammengestellt.

**Tabelle 4**

| Substrat | maximale Anfangs-Reakt.-Geschw. ($\mu$Mol x min$^{-1}$ x mg$^{-1}$) | $V_{max}K_M$-Wert (M) |
|---|---|---|
| 2-Keto-4-methylpentansäure | 64 | 0,07 x 10$^{-3}$ |
| 2-Ketopentansäure | 87 (6 x $K_M$) | 0,25 x 10$^{-3}$ |
| 2-Ketohexansäure | 69 (6 x $K_M$) | 0,12 x 10$^{-3}$ |
| 2-Keto-4-(methylmercapto)-buttersäure | 28 | 0,21 x 10$^{-3}$ |

Die Tabelle zeigt, daß eine Reihe von 2-Ketocarbonsäuren durch die erfindungsgemäße L-2-Hydroxy-4-methylpentansäure-Dehydrogenase zu den entsprechenden L-2-Hydroxy-carbonsäuren reduziert werden können.

Die in Tabelle 5 angegebenen relativen Aktivitäten (U/ml) wurden bei Substrat-Endkonzentrationen von 0,1 mM, 1 mM und 10 mM gemessen. Es wurde in allen Fällen die gleiche Enzymlösung eingesetzt. Bei 10 mM macht sich bei allen Substraten bereits eine Substratüberschuß-Inhibierung bemerkbar.

Die Abhängigkeit der Reaktionsgeschwindigkeit von der NADH-Konzentration wurde in einem Testansatz ermittelt, der 0,1 M Phosphatpuffer für pH 7,0, 0,79 mM 2-Keto-4-methylpentansäure und limitierende Mengen an Enzym enthielt. Die NADH-Konzentration wurde im Bereich von 0,01 bis 0,33 mM variiert. Der $K_M$-Wert für NADH wurde durch nicht-lineare Regression der Michaelis-Menten-Gleichung ermittelt und beträgt 3,3 x 10$^{-5}$ M.

**Tabelle 5**

| Substrat | Testkonzentration | | |
|---|---|---|---|
| | 0,1 mM (U/ml | 1 mM (U/ml | 10 mM (U/ml |
| 2-Keto-4-methylpentansäure | 133 | 259 | 144 |
| 2-Ketopentansäure | 149 | 344 | 161 |
| 2-Ketohexansäure | 123 | 301 | 103 |
| 2-Keto-4-(methylmercapto)-buttersäure | 33 | 93 | 53 |

Im nachfolgenden Beispiel 3 schließlich wird ein Versuch zur kontinuierlichen enzymatischen Umwandlung von 2-Keto-4-methylpentansäure in L-2-Hydroxy-4-methylpentansäure gemäß dem aus der US-PS 4 326 031 bekannten Verfahren beschrieben.

**Beispiel 3**

Ein auf einer Temperatur von 25°C gehaltener Flachmembranreaktor mit einem Volumen von 11,3 ml, der mit einem Magnetrührer und einer Ultrafiltrationsmembran von 62 mm Durchmesser mit einer nominellen Ausschlußgrenze von 5.000 (Lieferfirma: Amicon, Witten; Typ YM5) ausgestattet war, wurde zur Sterilisation mittels einer auf eine Fördergeschwindigkeit von 11,3 ml/Stunde eingestellten Dosierpumpe ca. 5 Stunden lang mit wässriger Formaldehydlösung durchgespült. Anschließend wurde während weiterer ca. 10 Stunden die Formaldehydlösung durch destilliertes Wasser verdrängt. Danach wurde, ebenfalls mit einer Fördergeschwindigkeit von 11,3 ml/Stunde, ca. 10 Stunden lang eine über ein Sterilfilter (0,2 µm) filtrierte Substratlösung zugeführt, die 100 mmol/l des Natriumsalzes der 2-Keto-4-methylpentansäure und 400 mmol/l

**0 103 210**

Natriumformiat sowie 50 mmol/l Kaliumphosphat als Puffer enthielt und mit Natronlauge auf pH 7 eingestellt war. Darauf wurden bei weiterlaufender Substratdosierung mit einer Spritze vor dem Sterilfilter 4 ml einer Lösung von Formiat-Dehydrogenase (Aktivität 26,3 µmol/ml x Minute bei Formiat als Substrat, 25°C und pH 8) in Form einer wässringen Lösung eingespritzt. Entsprechend wurden danach 0,5 ml L-2-Hydroxy-4-methylpentansäure-Dehydrogenase (Aktivität 241,7 µmol/ml x Minute bei 2-Keto-4-methylpentanoat als Substrat, 25°C und pH 7) in Form einer wässrigen Lösung eingespritzt. Zum Start der Reaktion wurden weiter eingespritzt 2 ml einer Coenzymlösung, die 5,73 mmol/l NADH, gebunden an ein Polyethylenglycol mit einem mittleren Molekulargewicht von 20.000, enthielt. Nachdem alle Katalysatoren eingespült waren, wurde der Substratstrom auf 17 ml/Stunde erhöht, so daß sich eine mittlere Verweilzeit von 40 Minuten ergab. Der Umsatz wurde kontinuierlich mit Hilfe einer in den Filtratstrom eeingebauten Polarimeterdurchflußküvette verfolgt. Der Differenzdruck über die Membran betrug zu Beginn 0,2 bar und stieg im Verlaufe von 8 Betriebstagen auf 1,3 bar an. Innerhalb einer Betriebszeit von 191 Stunden (ca. 8 Tagen) wurden 276 mmol, entsprechend 37 g L-2-Hydroxy-4-methylpentansäure erhalten. Der Umsatz fiel von 93,9 % auf 76,3 % ab (mittlerer Umsatz 85,1 %). Für das Coenzym ergab sich eine Desaktivierung von 9,29 % pro Tag, für die Formiat-Dehydrogenase von 4,54 % pro Tag, für die L-2-Hydroxy-4-methylpentansäure-Dehydrogenase 4,68 % pro Tag. Die Raum-Zeit-Ausbeute über die gesamte Betriebsdauer betrug im Mittel 3,07 mol/l x d bzw. 411 g/l x d. Pro mol Coenzym (verbraucht) wurden 71.800 mol Produkt gebildet. Das entspricht einem Bedarf von 68,9 mg $NAD^+$ (nativ)/kg Produkt. Der Enzymbedarf betrug 409 U Formiat-Dehydrogenase/kg Produkt bzw. 617 U L-2-Hydroxy-4-methyl-pentansäure-Dehydrogenase/kg Produkt. Die mittlere gemessene Formiat-Dehydrogenase-Aktivität während der gesamten Betriebszeit betrug 3,74 U/ml, bezüglich der 2-Hydroxy-4-methylpentansäure-Dehydrogenase 5,48 U/ml. Die mittlere Coenzymkonzentration betrug 0,48 mmol/l. Die mittlere Reaktionsgeschwindigkeit über die gesamte Betriebszeit betrug 2,13 µmol/ml x Minute.

**Patentansprüche**

1. L-2-Hydroxy-4-methylpentansäure-Dehydrogenase, gekennzeichnet durch die folgenden Eigenschaften:
a) spezifische Aktivität gegenüber L-2-Hydroxy-4-methylpentansäure,
b) abhängig von Nicotinamid-adenin-dinucleotid (NAD),
c) Temperatur-Optimum der Wirkung 40 bis 60°C,
d) Temperatur-Optimum der Stabilität $\leqslant$ 40°C,
e) pH-Optimum für die Dehydrogenierungsreaktion 8,0 bis 8,5,
f) pH-Optimum für die Reduktionsreaktion um 7,0,
g) pH-Stabilitätsbereich 6,5 bis 8,5,
h) Michaelis-Konstante ($K_M$-Wert) gegenüber L-2-Hydroxy-4-methylpentansäure 0,62 x $10^{-3}$M und
i) zusätzliche Aktivität gegenüber L-2-Hydroxy-pentansäure, L-2-Hydroxyhexansäure und L-2-Hydroxy-4-(methylmercapto)-buttersäure
und erhältlich durch Züchtung des Stammes Lactobacillus confusus DSM 20196 und Gewinnung daraus.

2. Verfahren zur Gewinnung der L-2-Hydroxy-4-methylpentan-säure-Dehydrogenase gemäß Anspruch 1, dadurch gekennzeichnet, daß man den Stamm Lactobacillus confusus DSM 20196 in einem wässrigen Nährmedium züchtet, das eine Quelle für Kohlenstoff und Stickstoff, sowie Mineralsalze, Wuchsstoffe und Vitamine enthält und zu Beginn der Züchtung einen pH von 6,5 aufweist, nach beendeter Züchtung die Zellen durch Zentrifugation erntet, sie in einer auf pH 7 gepufferten Suspension aufschließt und das Enzym aus dem Extrakt gewinnt.

3. Verwendung der L-2-Hydroxy-4-methylpentansäure-Dehydro-genase gemäß Anspruch 1 zur enzymatischen Umwandlung von L-2-Hydroxy-4-methylpentansäure, L-2-Hydroxypentan-säure, L-2-Hydroxyhexansäure oder L-2-Hydroxy-4-(methylmercapto)-buttersäure in die entsprechende 2-Ketocarbonsäure bzw. von 2-Keto-4-methylpentansäure, 2-Ketopentansäure, 2-Ketohexansäure oder 2-Keto-4-(methylmercapto)-buttersäure in die entsprechende L-2-Hydroxycarbonsäure.

**Claims**

1. L-2-hydroxy-4-methylpentanic acid dehydrogenase, characterised by the following properties:
a) specific activity towards L-2-hydroxy-4-methylpentanic acid,
b) dependent on nicotinamide adenine dinucleotide (NAD),
c) optimum temperature for effect 40 to 60°C,
d) optimum temperature for stability $\leqslant$ 40°C,
e) optimum pH for the dehydrogenation reaction 8.0 to 8.5,
f) optimum pH for the reduction reaction about 7.0,
g) pH stabilization range 6.5 to 8.5,
h) Michaelis constants ($K_M$-value) relative to L-2-hydroxy-4-methylpentanic acid 0.62 x $10^{-3}$M and
i) additional activity towards L-2-hydroxypentanic acid, L-2-hydroxyhexanic acid and L-2-hydroxy-4-(methyl-

6

mercapto)-butyric acid

and obtainable by culturing the strain <u>Lactobacillus confusus</u> DSM 20196 and recovery therefrom.

2. A process for the recovery of L-2-hydroxy-4-methyl-pentanic acid dehydrogenase according to claim 1, characterised in that the strain <u>Lactobacillus confusus</u> DSM 20196 is cultured in an aqueous nutrient containing a source of carbon and nitrogen as well as mineral salts, growth-promoting substances and vitamins and having a pH of 6.5 at the beginning of culturing, once the cells have been cultured they are gathered up by centrifugation, are broken up in a suspension buffered to pH 7 and the enzyme is recovered from the extract.

3. Use of L-2-hydroxy-4-methyl pentanic acid dehydrogenase according to claim 1 for the enzymatic conversion of L-2-hydroxy-4-methylpentanic acid, L-2-hydroxypentanic acid, L-2-hydroxyhexanic acid or L-2-hydroxy-4-(methylmercapto)-butyric acid into the corresponding 2-ketocarboxylic acid or of 2-keto-4-methylpentanic acid, 2-ketopentanic acid, 2-ketohexanic acid or 2-keto-4-(methylmercapto)-butyric acid into the corresponding L-2-hydroxycarboxylic acid.

## Revendications

1. Déhydrogènase de l'acide L-2-hydroxy-4-méthylvalérianique, caractérisée en ce qu'elle possède les propriétés suivantes:

a) activité spécifique vis-à-vis de l'acide L-2-hydroxy-4-méthylvalérianique,

b) dépendance envers le nicotinamide-adénine- dinucléotide (NAD)

c) température optimum d'action 40 à 60°C,

d) température optimum de stabilité $\leqslant$ 40°C,

e) pH optimum pour la réaction de déshydrogènation, 8 à 8,5,

f) pH optimum pour la réaction de réduction, vers 7,

g) pH du champ de stabilisation, 6,5 à 8,5,

h) constante de Michaelis (valeur $K_M$) par rapport à l'acide L-2-hydroxyxy-4-méthylvalérianique, $0,62 \times 10^{-3}M$; et

i) activité supplémentaire vis-à-vis de l'acide L-2-hydroxy-valérianique, de l'acide L-2-hydroxycaproïque et de l'acide L-2-hydroxy-4- (méthylmercapto)-butyrique, pouvant être obtenue par culture de la souche Lactobacillus confusus DSM 20 196 et récupération à partir de cette culture.

2. Procédé pour l'obtention de la déhydrogènase de l'acide L-2-hydroxy-4-méthylvalérianique suivant la revendication 1, caractérisé en ce que l'on cultive la souche de Lactobacillus confusus DSM 20 196 dans un milieu nutritif aqueux, qui contient une source de carbone et d'azote, ainsi que des sels minéraux, des substances de croissance et des vitamines, et qui présente, au commencement de la culture, un pH de 6,5, que, la culture une fois terminée, on récolte les cellules par centrifugation, les ouvre dans une suspension tamponnée à pH 7, et récupère l'enzyme à partir de l'extrait.

3. Utilisation de la déhydrogènase de l'acide L-2-hydroxy-4-méthylvalérianique suivant la revendication 1 pour la conversion des acides L-2-hydroxy-4-méthylvalérianique, L-2-hydroxyvalérianique, L-2-hydroxy-caproïque, ou L-2-hydroxy-4-(méthylmercapto)-butyrique en les acides 2-cétocarboxyliques correspondants, ou encore, des acides 2-céto-4-méthylvalérianique, 2-céto-valérianique, 2-cétocaproïque, ou 2-céto-4-(méthylmercapto)-butyrique en les acides L-2-hydroxycarboxyliques correspondants.